# EUROPEAN PATENT SPECIFICATION

(11) **EP 4 175 653 B1**
(45) Date of publication and mention of the grant of the patent: **31.12.2025**
(21) Application number: 21890438.1
(22) Date of filing: 11.11.2021
(51) Int. Cl.: A61K 31/7024, A61K 31/235, A61K 31/14, A61K 36/22, A61K 36/54, A61K 36/61, A61K 9/20, A61K 9/48, A61K 35/644, A61P 31/12, A61P 37/04

(54) **COMPOSITIONS COMPRISING NATURAL EXTRACTS FOR STIMULATING THE IMMUNE RESPONSE**
ZUSAMMENSETZUNGEN MIT NATÜRLICHEN EXTRAKTEN ZUR STIMULIERUNG DER IMMUNREAKTION
COMPOSITIONS COMPRENANT DES EXTRAITS NATURELS POUR STIMULER LA RÉPONSE IMMUNITAIRE

(30) Priority: 13.11.2020 US 202063113319 P
(43) Date of publication of application: 10.05.2023
(73) Proprietor: Shiban Canada Inc., Laval QC H7M 2R7 (CA)
(72) Inventor: AL-SHAIBANI, Abdulhameed Abdulridha K., Beirut (LB); MAALLAH, Abderrahim, Laval, Québec H7M 2R7 (CA)
(74) Representative: Cabinet Becker et Associés
(86) International application number: PCT/CA2021/051604
(87) International publication number: WO 2022/099414

(56) References cited:
- CN-A- 1 466 987
- CN-A- 102 631 373
- CN-A- 104 920 772
- CN-A- 105 687 226
- CN-A- 108 967 692
- CN-A- 110 643 471
- CN-A- 111 759 851
- DE-A1- 19 904 801
- US-A1- 2019 008 907
- ANJUM SYED ISHTIAQ ET AL: "Composition and functional properties of propolis (bee glue): A review", SAUDI JOURNAL OF BIOLOGICAL SCIENCES, ELSEVIER, AMSTERDAM, NL, vol. 26, no. 7, 17 August 2018 (2018-08-17), pages 1695 - 1703, XP085909024, ISSN: 1319-562X, [retrieved on 20180817], DOI: 10.1016/J.SJBS.2018.08.013
- MAYWORM MARCO A. S. ET AL: "Does Propolis Contain Tannins?", vol. 2014, 14 May 2014 (2014-05-14), US, pages 1 - 4, XP093146463, ISSN: 1741-427X, Retrieved from the Internet <URL:https://www.ncbi.nlm.nih.gov/pmc/articles/PMC4052103/pdf/ECAM2014-613647.pdf> DOI: 10.1155/2014/613647
- BERRETTA ANDRESA APARECIDA; SILVEIRA MARCELO AUGUSTO DUARTE; CóNDOR CAPCHA JOSé MANUEL; DE JONG DAVID: "Propolis and its potential against SARS-CoV-2 infection mechanisms and COVID-19 disease Running title: Propolis against SARS-CoV-2 infection and COVID-19", BIOMEDICINE & PHARMACOTHERAPY, ELSEVIER, FR, vol. 131, 17 August 2020 (2020-08-17), FR , XP086322072, ISSN: 0753-3322, DOI: 10.1016/j.biopha.2020.110622
- KORKMAZ HASAN: "Could Sumac Be Effective on COVID-19 Treatment?", JOURNAL OF MEDICINAL FOOD, MARY ANN LIEBERT, LARCHMONT, NY,, US, vol. 24, no. 6, 1 June 2021 (2021-06-01), US , pages 563 - 568, XP055938545, ISSN: 1096-620X, DOI: 10.1089/jmf.2020.0104

## Description

### TECHNICAL FIELD

The present invention generally relates to the field of immunology, and the enhancement of the immune response against pathogens such as viruses. It relates to a composition comprising propolis and tannic acid.

### BACKGROUND ART

The immune system protects the body from outside invaders, such as bacteria, viruses, fungi, and toxins, as well as against cancers.

The innate immune response is the first line of defense against viral infections, which is rapid in response, but nonspecific. During the infection, conserved components from the pathogen called pathogen associated molecular patterns (PAMPs) are recognized by host pathogen recognition receptors (PRRs), such as retinoic acid-inducible gene-I protein (RIG-I) and toll-like receptor (TLR), leading to activation of innate immune signaling that finally induces the production of various cytokines and antiviral molecules such as interferons (IFNs) and pro-inflammatory cytokines such as Tumor Necrosis Factor (TNF), Interleukin (IL)-6, and IL-1β. These PAMPs have certain characteristic of viral RNA that are not shared by cellular RNAs, such as regions of double-stranded RNA (dsRNA) or the presence of a 5'-triphosphate group.

T cells and B cells play key roles in adaptive immunity against viral infections. T cells are mainly known as CD4⁺ T and CD8⁺ T cells. CD8⁺ T cells differentiate into cytotoxic T lymphocytes (CTLs), which produce cytokines and effector molecules to restrict viral replication and kill virus-infected cells.

Immunodeficiencies occur when one or more of the components of the immune system are inactive. The ability of the immune system to respond to pathogens is diminished in both the young and the elderly, with immune responses beginning to decline at around 50 years of age due to immunosenescence. In developed countries, obesity, alcoholism, and drug use are common causes of poor immune function, while malnutrition is the most common cause of immunodeficiency in developing countries. Diets lacking sufficient protein are associated with impaired cell-mediated immunity, complement activity, phagocyte function, IgA antibody concentrations, and cytokine production. Additionally, the loss of the thymus at an early age through genetic mutation or surgical removal results in immunodeficiency and a high susceptibility to infection.

The coronavirus disease 2019 (COVID-19) pandemic, also known as the coronavirus pandemic, is an ongoing global pandemic caused by severe acute respiratory syndrome coronavirus 2 (SARS-CoV-2), first identified in December 2019 in Wuhan, China. The World Health Organisation (WHO) declared the outbreak a Public Health Emergency of International Concern in January 2020 and a pandemic in March 2020. As of November 11, 2020, more than 51.5 million cases have been confirmed, with more than 1.27 million deaths attributed to COVID-19. Common symptoms include fever, cough, fatigue, breathing difficulties, and loss of smell and taste. Complications may include pneumonia and acute respiratory distress syndrome.

Current evidence indicates that SARS-CoV-2, the etiologic agent of COVID-19, will become endemic in the population. The current pandemic is aggravated by the apparition of variants of concern that are feared to result in an antigenic drift that could evade vaccine-elicited immune responses.

CN111759851 describes a spray composition and an oral formulation comprising tannic acid for the treatment of a SARS-CoV 2 infection. CN1466987 describes tablets comprising propolis for use in preventing SARS and influenza infection.

Given the importance of the immune system in the defense against infections and related diseases such as COVID-19, there is a need for novel approaches for stimulating or boosting the immune system, especially in subjects with immunodeficiencies such as elderly people.

### SUMMARY

The present disclosure is as defined in claims 1-15.

More particularly, it relates to a composition comprising from 20% to about 75% (w/w) of propolis and from 10% to 30% (w/w) of tannic acid. Preferably, the composition comprises from about 25% to about 70% (w/w) of propolis. Preferably, the composition comprises from about 10% to about 25% (w/w) of tannic acid.

Optionally, the composition further comprises gum arabic, cinnamon and/or clove. Optionally, the composition comprises about 10% to about 30% (w/w) of gum arabic. Optionally, the composition comprises about 10% to about 30% (w/w) of cinnamon. Optionally, the composition further comprises about 1% to about 10% (w/w) of clove.

Optionally, the composition further comprises at least one carrier or excipient, especially a binder such as a cellulose-based binder, and/or a lubricant such as magnesium stearate.

In a particular aspect, the composition comprises:

| Ingredients | % (w/w) |
|---|---|
| Propolis | 50-70% |
| Tannic acid | 20-30% |
| Magnesium stearate | 0.5-2% |
| Microcrystalline cellulose | 5-15%. |

In another particular aspect, the composition comprises:

| Ingredients | % (w/w) |
|---|---|
| Propolis | 20-35% |
| Tannic acid | 14-20% |
| Gum arabic | 15-27% |
| Cinnamon powder | 10-20% |
| Clove powder | 2-8% |
| Magnesium stearate | 0.5-2% |
| Microcrystalline cellulose | 5-15%. |

The present invention relates to an oral dosage form comprising the composition as claimed. The present invention relates to the composition or the dosage form as claimed for use in treating an infection in a subject. Optionally, the infection is an influenza infection, an RSV infection or a SARS-CoV-2 infection.

Other objects, advantages and features of the present disclosure will become more apparent upon reading of the following description of specific aspects thereof, given by way of example only with reference to the accompanying drawings.

### BRIEF DESCRIPTION OF DRAWINGS

In the appended drawings:

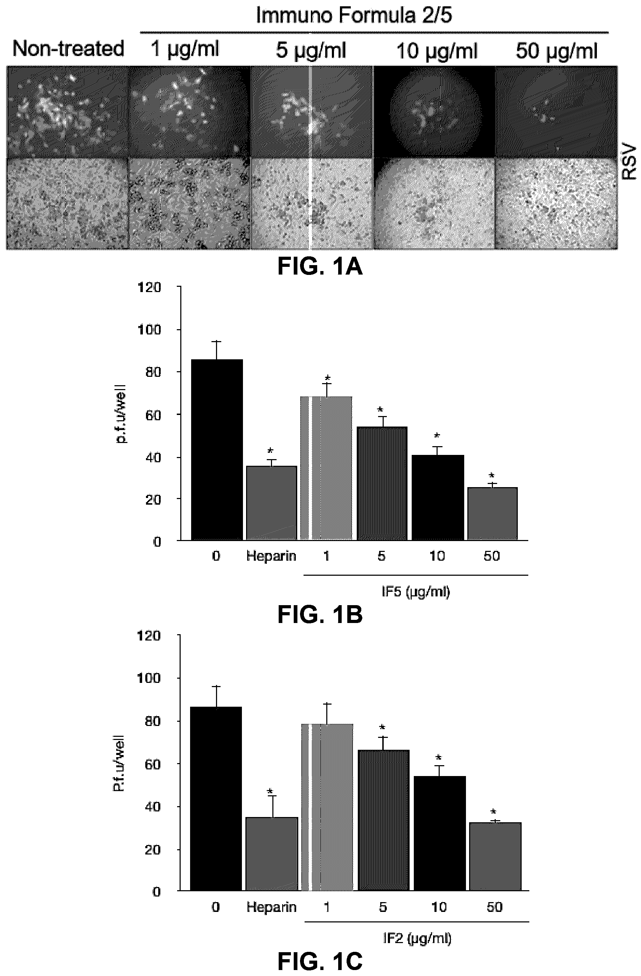

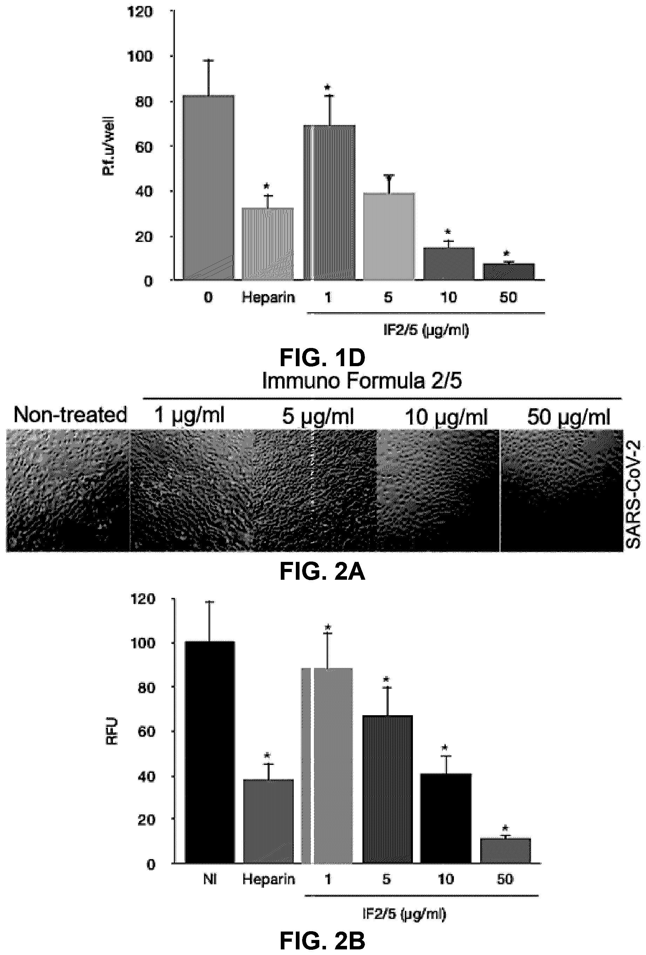

**FIGs. 1A-D** show the antiviral activities of the formulations against RSV *in vitro.* **FIG. 1A****:** Fluorescent images of A549 cells infected with RSV-GFP at a multiplicity of infection (MOI) of 2, containing a serially diluted mixture of formulation 1 and formulation 2 (Immuno Formula 2/5) as described in Example 3. At 48 h pi, cells were washed, fixed and examined by a confocal microscopy. **FIGs. 1B****-D:** RSV plaque-forming units per well detected 6 days post-infection in A549 cells treated and untreated with formulation 1 (IF5, **FIG**. **1B**), formulation 1 (IF2, **FIG**. **1C**), or a mixture of IF5 and IF2 (IF2/5, **FIG. 1D**) and exposed to RSV. Cell monolayers were stained with Crystal violet after 6 days of infection at 37°C. Heparin was used as a positive control. Data represent mean values of at least three independent experiments, and error bars indicate SEM. *P<0.05 versus non-treated cells; unpaired t-test.
**FIGs. 2A-B** show the antiviral activities of the formulations against SARS-CoV-2 pseudovirus *in vitro.* **FIG. 2A****:** Fluorescent images of A549 cells infected with SARS-CoV-2-GFP at a multiplicity of infection (MOI) of 1, containing a serially diluted mixture of formulation 1 and formulation 2 (Immuno Formula 2/5) as described in Example 3. At 48 h pi, cells were washed, fixed and examined by a confocal microscopy. **FIG. 2B**: A549 cells were infected with SARS-CoV-2 at an MOI of 2 containing a serially diluted mixture of formulation 1 and formulation 2 (IF2/5). At 48 h pi, SARS-CoV-2-GFP green fluorescence was measured. The results are expressed as mean ± s.d for three independent experiments. Heparin was used as a positive control. Data represent mean values of at least three independent experiments, and error bars indicate SEM. P<0.05 versus non-treated cells; unpaired t-test. NI, Non-infected. RFU, Relative fluorescence units.

### DETAILED DISCLOSURE

The use of the terms "a" and "an" and "the" and similar referents in the context of describing the technology (especially in the context of the following claims) are to be construed to cover both the singular and the plural, unless otherwise indicated herein or clearly contradicted by context.

The terms "comprising", "having", "including", and "containing" are to be construed as openended terms (i.e., meaning "including, but not limited to") unless otherwise noted.

All methods described herein can be performed in any suitable order unless otherwise indicated herein or otherwise clearly contradicted by context.

Herein, the term "about" has its ordinary meaning. The term "about" is used to indicate that a value includes an inherent variation of error for the device or the method being employed to determine the value, or encompass values close to the recited values, for example within 10% of the recited values (or range of values).

Unless specifically defined otherwise, all technical and scientific terms used herein shall be taken to have the same meaning as commonly understood by one of ordinary skill in the art (*e.g.,* in the pharmaceutical or drug formulation).

The present inventors have developed compositions comprising a mixture of ingredients that have complementary/synergistic antimicrobial and/or immune-stimulating effects. These compositions were shown to exhibit antiviral properties in *in vitro* models of RSV and SARS-CoV-2 infections.

The present disclosure provides a composition comprising from 20% to about 75% (w/w) of propolis and from 10% to 30% (w/w) of tannic acid (*e.g.,* sumac powder). The skilled person would understand that the proportion of the two ingredients cannot exceed 100%, and thus, *e.g.,* if the proportion of propolis is 75%, the proportion of tannic acid cannot exceed 25%.

Propolis (also sometimes referred to as "bee glue") is a resinous mixture produced by honeybees, and which comprises a mixture of saliva and beeswax with exudate gathered from tree buds (*e.g.,* from birch, poplar, pine, alder, willow, palm, *Baccharis dracunculifolia,* and *Dalbergia ecastaphyllum*)*,* sap flows, or other botanical sources such as flowers and exudates of plants. Propolis typically comprises flavonoids, phenolics, and aromatic compounds such as kaempferol, quercetin, galangin, chrysin, pinocembrine, coumaric acid, 3,5 Diprenyl-*p*-coumaric (artepellin C) and saccharin.

In an aspect, the composition comprises from about 25% to about 70% (w/w) of propolis. In another aspect, the composition comprises from about 20% to about 40%, from about 20% to about 35%, from about 25% to about 35%, or from about 25% to about 30%, of propolis. In another aspect, the composition comprises from about 40% to about 75%, from about 50% to about 75%, from about 55% to about 65%, from about 60% to about 65%, or from about 61% to about 64%, of propolis.

Tannic acid is a polyphenol of the following chemical structure:

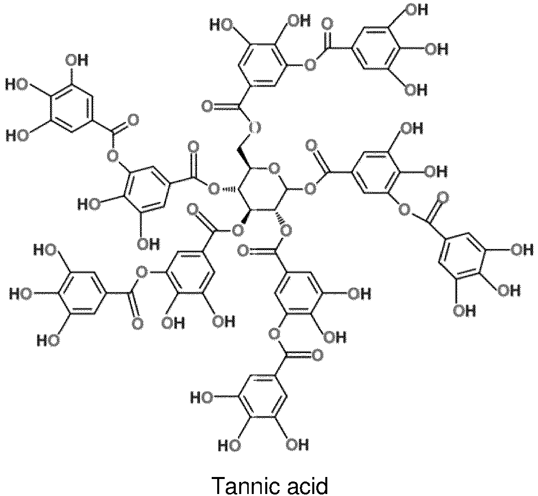

Tannic acid is found in two forms, quercitannic acid that is mainly found in oak bark and leaves, and gallotannic acid that is mainly found in oak galls. Tannic acid is found at high levels in sumac powder.

In an aspect, the composition comprises from about 10% to about 25%, from about 10% to about 20%, from about 12% to about 20%, or from about 14% to about 18%, of tannic acid. In another aspect, the composition comprises from about 15% to about 30%, from about 20% to about 30%, from about 23% to about 27%, or from about 24% to about 26%, of tannic acid. In an aspect, the tannic acid is comprised in sumac powder. Thus, in aspects, the composition comprises from about 10% to about 25%, from about 10% to about 20%, from about 12% to about 20%, or from about 14% to about 18%, of sumac powder. In another aspect, the composition comprises from about 15% to about 30%, from about 20% to about 30%, from about 23% to about 27%, or from about 24% to about 26%, of sumac powder.

In another aspect, the composition further comprises cinnamon and/or clove, more particularly cinnamon powder and/or clove powder.

In an aspect, the composition comprises cinnamon powder. In an aspect, the composition comprises about 10% to about 30% of cinnamon powder. In another aspect, the composition comprises from about 10% to about 25%, from about 10% to about 20%, from about 12% to about 20%, or from about 14% to about 18%, of cinnamon powder. Cinnamon has total phenolic and flavonoid contents of 269 mg/100 g and 62 mg/100 g, respectively, and has good antioxidant capacities as determined using DPPH and ABTS assays.

In another aspect, the composition further comprises clove, more particularly clove powder. In an aspect, the composition comprises about 1% to about 10% of clove powder. In another aspect, the composition comprises from about 2% to about 8%, from about 3% to about 7%, from about 4% to about 6%, or from about 5% to about 6%, of clove powder. Clove is considered to have protective effects against degenerative diseases, including cancer.

In another aspect, the composition further comprises gum arabic (acacia gum). In an aspect, the composition comprises about 10% to about 30% of gum arabic. In another aspect, the composition comprises from about 10% to about 25%, from about 15% to about 25%, from about 20% to about 25%, or from about 21% to about 23%, of gum arabic. Gum arabic contains high levels of flavonoid and polyphenol antioxidants. *A. arabica* extract has hypoglycemic, anti hyperlipidemic, and antioxidant properties in streptozotocin-induced diabetic rats.

In another aspect, the composition further comprises at least one carrier or excipient, in a further aspect a pharmaceutically acceptable carrier or excipient. Such compositions may be prepared in a manner well known in the pharmaceutical art (see Remington: The Science and Practice of Pharmacy, by Loyd V Allen, Jr, 2012, 22nd edition, Pharmaceutical Press; Handbook of Pharmaceutical Excipients, by Rowe et al., 2012, 7th edition, Pharmaceutical Press).

An "excipient," as used herein, has its normal meaning in the art and is any ingredient that is not an active ingredient (drug) itself. Excipients include for example binders, lubricants, diluents, fillers, thickening agents, disintegrants, plasticizers, coatings, barrier layer formulations, lubricants, stabilizing agent, release-delaying agents and other components. "Pharmaceutically acceptable excipient" as used herein refers to any excipient that does not interfere with effectiveness of the biological activity of the active ingredients and that is not toxic to the subject, *i.e.,* is a type of excipient and/or is for use in an amount which is not toxic to the subject. Excipients are well known in the art, and the present system is not limited in these respects. In certain aspects, the composition includes excipients, including for example and without limitation, one or more binders (binding agents), thickening agents, surfactants, diluents, release-delaying agents, colorants, flavoring agents, fillers, disintegrants/dissolution promoting agents, lubricants, plasticizers, silica flow conditioners, glidants, anti-caking agents, anti-tacking agents, stabilizing agents, anti-static agents, swelling agents and any combinations thereof. As those of skill would recognize, a single excipient can fulfill more than two functions at once, *e.g.,* can act as both a binding agent and a thickening agent. As those of skill will also recognize, these terms are not necessarily mutually exclusive.

Useful diluents, *e.g.,* fillers, include, for example and without limitation, dicalcium phosphate, calcium diphosphate, calcium carbonate, calcium sulfate, lactose, cellulose, kaolin, sodium chloride, starches, powdered sugar, colloidal silicon dioxide, titanium oxide, alumina, talc, colloidal silica, microcrystalline cellulose, silicified micro crystalline cellulose and combinations thereof. Fillers that can add bulk to tablets with minimal drug dosage to produce tablets of adequate size and weight include croscarmellose sodium NF/EP (*e.g.,* Ac-Di-Sol); anhydrous lactose NF/EP (*e.g*., Pharmatose^{™} DCL 21); and/or povidone USP/EP.

Binder materials include, for example and without limitation, starches (including corn starch and pregelatinized starch), gelatin, sugars (including sucrose, glucose, dextrose and lactose), polyethylene glycol, povidone, waxes, and natural and synthetic gums, *e.g.,* acacia sodium alginate, polyvinylpyrrolidone, cellulosic polymers (*e.g.,* hydroxypropyl cellulose, hydroxypropyl methylcellulose, methyl cellulose, hydroxyethyl cellulose, carboxymethylcellulose, colloidal silicon dioxide NF/EP (*e.g.,* Cab-O-Sil^{™} M5P), Silicified Microcrystalline Cellulose (SMCC), *e.g.,* Silicified microcrystalline cellulose NF/EP (*e.g*., Prosolv^{™} SMCC 90, COMPRECEL^{®}), and silicon dioxide, mixtures thereof, and the like), veegum, and combinations thereof. In an aspect, the composition comprises a binder. In an aspect, the composition comprises from about 1% to about 20% (w/w) of binder. In further aspects, the composition comprises from about 2% to about 20%, from about 4% to about 15%, from about 5% to about 15%, from about 8% to about 14%, from about 10% to about 14%, or about 10%, about 10.5%, about 11%, about 11.5%, about 12%, about 12.5% or about 13% (w/w), of binder. In an aspect, the binder comprises or is Silicified Microcrystalline Cellulose.

Useful lubricants include, for example, canola oil, glyceryl palmitostearate, hydrogenated vegetable oil (type I), magnesium oxide, magnesium stearate, mineral oil, poloxamer, polyethylene glycol, sodium lauryl sulfate, sodium stearate fumarate, stearic acid, talc and, zinc stearate, glyceryl behapate, magnesium lauryl sulfate, boric acid, sodium benzoate, sodium acetate, sodium benzoate/sodium acetate (in combination), DL-leucine, calcium stearate, sodium stearyl fumarate, mixtures thereof, and the like. In an aspect, the composition comprises a lubricant. In an aspect, the composition comprises from about 0.05% to about 3% (w/w) of lubricant. In further aspects, the composition comprises from about 0.1% to about 2%, from about 0.1% to about 1.5%, from about 0.1% to about 1.0%, from about 0.5% to about 1.0%, or about 0.5%, about 0.6%, about 0.7%, about 0.8%, about 0.9% or about 1% (w/w), of lubricant. In an aspect, the lubricant comprises or is magnesium stearate.

Bulking agents include, for example: microcrystalline cellulose, for example, AVICEL^{®} (FMC Corp.), COMPRECEL^{®}, EMCOCEL^{®} (Mendell Inc.), which also has binder properties; dicalcium phosphate, for example, EMCOMPRESS^{®} (Mendell Inc.); calcium sulfate, for example, COMPACTROL^{®} (Mendell Inc.); and starches, for example, Starch 1500; and polyethylene glycols (CARBOWAX^{®}).

Disintegrating or dissolution promoting agents include: starches, clays, celluloses, alginates, gums, crosslinked polymers, colloidal silicon dioxide, osmogens, mixtures thereof, and the like, such as crosslinked sodium carboxymethyl cellulose (AC-DI-SOL^{®}), sodium croscarmelose, sodium starch glycolate (EXPLOTAB^{®}, PRIMO JEL^{®}) crosslinked polyvinylpolypyrrolidone (PLASONE-XL^{®}), sodium chloride, sucrose, lactose and mannitol.

Antiadherents and glidants employable in the core and/or a coating of the solid oral dosage form may include talc, starches (*e.g.,* cornstarch), celluloses, silicon dioxide, sodium lauryl sulfate, colloidal silica dioxide, and metallic stearates, among others.

Examples of silica flow conditioners include colloidal silicon dioxide, magnesium aluminum silicate and guar gum.

Suitable surfactants include pharmaceutically acceptable non-ionic, ionic and anionic surfactants. An example of a surfactant is sodium lauryl sulfate. If desired, the pharmaceutical composition to be administered may also contain minor amounts of nontoxic auxiliary substances such as wetting or emulsifying agents, pH-buffering agents and the like, for example, sodium acetate, sorbitan monolaurate, triethanolamine sodium acetate, triethanolamine oleate, etc. If desired, flavoring, coloring and/or sweetening agents may be added as well.

Examples of stabilizing agents include acacia, albumin, polyvinyl alcohol, alginic acid, bentonite, dicalcium phosphate, carboxymethylcellulose, hydroxypropylcellulose, colloidal silicon dioxide, cyclodextrins, glyceryl monostearate, hydroxypropyl methylcellulose, magnesium trisilicate, magnesium aluminum silicate, propylene glycol, propylene glycol alginate, sodium alginate, carnauba wax, xanthan gum, starch, stearate(s), stearic acid, stearic monoglyceride and stearyl alcohol.

Examples of thickening agent can be for example talc USP/EP, a natural gum, such as guar gum, or a cellulose derivative such as microcrystalline cellulose NF/EP (e.g., Avicel^{™} PH 102), methylcellulose, ethylcellulose or hydroxyethylcellulose. A useful thickening agent is hydroxypropyl methylcellulose, an adjuvant which is available in various viscosity grades.

Examples of plasticizers include: acetylated monoglycerides; these can be used as food additives; Alkyl citrates, used in food packagings, medical products, cosmetics and children toys; Triethyl citrate (TEC); Acetyl triethyl citrate (ATEC), higher boiling point and lower volatility than TEC; Tributyl citrate (TBC); Acetyl tributyl citrate (ATBC), compatible with PVC and vinyl chloride copolymers; Trioctyl citrate (TOC), also used for gums and controlled release medicines; Acetyl trioctyl citrate (ATOC), also used for printing ink; Trihexyl citrate (THC), compatible with PVC, also used for controlled release medicines; Acetyl trihexyl citrate (ATHC), compatible with PVC; Butyryl trihexyl citrate (BTHC, trihexyl o-butyryl citrate), compatible with PVC; Trimethyl citrate (TMC), compatible with PVC; alkyl sulphonic acid phenyl ester, polyethylene glycol (PEG) or any combination thereof. Optionally, the plasticizer can comprise triethyl citrate NF/EP.

Examples of permeation enhancers include: sulphoxides (such as dimethylsulphoxide, DMSO), azones (e.g. laurocapram), pyrrolidones (for example 2-pyrrolidone, 2P), alcohols and alkanols (ethanol, or decanol), glycols (for example propylene glycol and polyethylene glycol), surfactants and terpenes.

In an aspect, the composition comprises the following ingredients:

| **Ingredients** | **% (w/w)** |
|---|---|
| Propolis | 50-70% |
| Tannic acid | 20-30% |
| Magnesium stearate | 0.5-2% |
| Microcrystalline cellulose | 5-15% |

In a further aspect, the composition comprises the following ingredients:

| **Ingredients** | **% (w/w)** |
|---|---|
| Propolis | 60-65%, *e.g.,* 62.5% |
| Tannic acid | 23-27%, *e.g.,* 25% |
| Magnesium stearate | 0.5-1%, *e.g.,* 0.75% |
| Microcrystalline cellulose | 10-13%, *e.g.,* 11.75% |

In another aspect, the composition comprises the following ingredients:

| **Ingredients** | **% (w/w)** |
|---|---|
| Propolis | 20-35% |
| Tannic acid | 14-20% |
| Gum arabic | 15-27% |
| Cinnamon powder | 10-20% |
| Clove powder | 2-8% |
| Magnesium stearate | 0.5-2% |
| Microcrystalline cellulose | 5-15% |

In a further aspect, the composition comprises the following ingredients:

| **Ingredients** | **% (w/w)** |
|---|---|
| Propolis | 25-30%, *e.g.,* 27,8% |
| Tannic acid | 15-18%, *e.g.,* 16.7% |
| Gum arabic | 20-24%, *e.g.,* 22.2% |
| Cinnamon powder | 13-18%, *e.g.,* 16.7% |
| Clove powder | 4-7%, *e.g.,* 5.6% |
| Magnesium stearate | 0.5-1%, *e.g.,* 0.7% |
| Microcrystalline cellulose | 9-12%, *e.g.,* 10.4% |

In an aspect, the composition is formulated for oral administration, e.g., is an oral dosage form. Formulations suitable for oral administration may include (a) liquid solutions, such as an effective amount of active agent(s)/composition(s) suspended in diluents, such as water, saline or PEG 400; (b) capsules, sachets or tablets, each containing a predetermined amount of the active ingredient, as liquids, solids, granules or gelatin; (c) suspensions in an appropriate liquid; and (d) suitable emulsions. Tablet forms can include one or more of lactose, sucrose, mannitol, sorbitol, calcium phosphates, corn starch, potato starch, microcrystalline cellulose, gelatin, colloidal silicon dioxide, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise the active ingredient in a flavor, *e.g.,* sucrose, as well as pastilles comprising the active ingredient in an inert base, such as gelatin and glycerin or sucrose and acacia emulsions, gels, and the like containing, in addition to the active ingredient(s), carriers known in the art.

In an aspect, the composition described herein is encapsulated. Thus, in another aspect, the present disclosure provides a capsule comprising the composition described herein. In an aspect, the capsule is a hard-shelled capsule. The capsule may be made of any material commonly used in the drug and/or food industry. Example of materials suitable for capsules include gelling agents, such as animal protein (mainly gelatin) or plant polysaccharides or their derivatives (such as carrageenan and modified forms of starch and cellulose such as hypromellose). Other ingredients can be added to the gelling agent including plasticizers such as glycerin or sorbitol to decrease the capsule's hardness, coloring agents, preservatives, disintegrants and/or lubricants. In an aspect, the capsule is a hard gelatin capsule. The capsule may be of any size (i.e., size #000 to #5), which is selected according to the desired amount of composition to be encapsulated. In an aspect, the size of the capsule is preferably size #000 to #0, for example size #00. The process of encapsulation of hard capsules such as hard gelatin capsules may be done on manual, semi-automatic and automatic apparatuses, which are well known in the art.

Thus, in another aspect, the present disclosure provides a dosage form, such as an oral dosage form, comprising the composition described herein. The term "dosage form" as used herein refers to a product, such as a pharmaceutical drug product, in the form in which it is marketed for use, i.e., in a particular configuration (*e.g.,* capsule shell, tablet), and apportioned into a particular dose. In an aspect, the dosage form is an oral dosage form, for example a capsule.

The dosage form may comprise any suitable amount of the composition described herein. In an aspect, the dosage form comprises from about 500 mg to about 2000 mg of the composition described herein, in further aspects from about 600 mg to about 1500 mg, from about 700 mg to about 1200 mg, from about 800 mg to about 1000 mg, or from about 800 mg to about 900 mg.

In an aspect, the dosage form comprises about 400 to about 600 mg or about 450 to about 550 mg of propolis, and about 100 to 300 mg or about 150 to 250 mg of tannic acid. In a further aspect, the dosage form comprises about 475 to about 525 or about 500 mg of propolis, and about 175 to about 225 mg or about 200 mg of tannic acid.

In another aspect, the dosage form comprises about 150 to about 350 or about 200 to 300 mg of propolis; about 50 to 250 mg or about 100 to 200 mg of tannic acid; about 100 to about 300 mg or about 150 to about 250 mg of gum arabic; about 50 to 250 mg or about 100 to 200 mg of cinnamon powder; and about 10 to about 100 mg or about 20 to about 90 mg of clove powder. In a further aspect, the dosage form comprises about 225 to about 275 or about 250 mg of propolis; about 125 to 175 mg or about 150 mg of tannic acid; about 175 to about 225 mg or about 200 mg of gum arabic; about 125 to 175 mg or about 150 mg of cinnamon powder; and about 40 to about 60 mg or about 50 mg of clove powder.

In another aspect, the present disclosure provides the composition or dosage form described herein for use in enhancing or stimulating the immune response in a subject.

The expression "enhancing or stimulating the immune response in a subject" means that the subject is able to mount a stronger immune response against infectious agents and/or tumors after administration of the composition or dosage form.

In another aspect, the present disclosure provides the composition or dosage form described herein for use in preventing or treating an infection and/or an infectious disease in a subject.

The subject may be a child or an adult. The subject may be an immunocompromised subject, for example a subject receiving immunosuppressive therapy. In an aspect, the subject is an elderly subject. In an aspect, the subject suffers from an infection, or is at risk of suffering from an infection

The term "infection" in the context of the present disclosure means an infection by a pathogenic microorganism, such as bacteria, fungus or virus. In an aspect, the infection is an infection in the respiratory tract, such as the upper or lower respiratory tract, caused by a pathogen, such as a virus or bacteria. The viral infection in the respiratory tract may be a human coronavirus infection or an influenza virus infection. Other viral infections may be caused by a picornavirus (*e.g.,* rhinovirus), human parainfluenza virus, human respiratory syncytial virus, adenovirus, enterovirus, or metapneumovirus. The bacterial infection of the respiratory tract may be caused by bacteria such as *Chlamydia pneumoniae, Streptococcus pneumoniae, Streptococcus pyrogenes, Haemophilus influenza, Moraxella catarrhalis,* or a mycobacterium such as M. *tuberculosis, M. bovis, M. africanum, M. canetti, M. microti.,* and *Burkholderia Sp.*

The "prevention" of a pathogenic infection, condition or disease refers to the reduction of the occurrence of the infection, condition or disease in the treated subject relative to an untreated subject, or delays the onset or reduces the severity of one or more symptoms of the infection, condition or disease relative to the untreated control subject.

In a further aspect, the subject suffers from or is at risk of suffering from a viral infection, for example a viral infection of the respiratory tract, such as an influenza infection, an RSV infection or a SARS-CoV-2 infection (COVID-19).

Any suitable amount of the composition or dosage form may be administered to a subject. The dosages will depend on many factors including the mode of administration. Typically, the amount of the composition contained within a single dose will be an amount that effectively enhances or stimulates the immune response in a subject, or that effectively prevents, delays or treats an infection in a subject, without inducing significant toxicity.

For the prevention, treatment or reduction in the severity of a given disease or condition, the appropriate dosage of the compound/composition will depend on the type of disease or condition to be treated, the severity and course of the disease or condition, whether the compound/composition is administered for preventive or therapeutic purposes, previous therapy, the patient's clinical history and response to the compound/composition, and the discretion of the attending physician. The compound/composition is suitably administered to the patient at one time or over a series of treatments. Preferably, it is desirable to determine the dose-response curve *in vitro,* and then in useful animal models prior to testing in humans. For example, depending on the type and severity of the disease and the patient, about 1 µg/kg to to 1000 mg per kg (mg/kg) of body weight per day. Further, the effective dose may be 0.5 mg/kg, 1 mg/kg, 5 mg/kg, 10 mg/kg, 15 mg/kg, 20 mg/kg/ 25 mg/kg, 30 mg/kg, 35 mg/kg, 40 mg/kg, 45 mg/kg, 50 mg/kg, 55 mg/kg, 60 mg/kg, 70 mg/kg, 75 mg/kg, 80 mg/kg, 90 mg/kg, 100 mg/kg, 125 mg/kg, 150 mg/kg, 175 mg/kg, 200 mg/kg, and may increase by 25 mg/kg increments up to 1000 mg/kg, or may range between any two of the foregoing values. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

The composition or dosage form described herein may be administered according to any suitable dosage regimen. In an aspect, the composition or dosage form is administered or is for administration three times-a-day. In an aspect, the composition or dosage form is administered or is for administration twice-a-day. In an aspect, the composition or dosage form is administered or is for administration once-a-day. In an aspect, the composition or dosage form is administered or is for administration once every two days. In an aspect, the composition or dosage form is administered or is for administration once every three days. In an aspect, the composition or dosage form is administered or is for administration twice-a-week. In an aspect, the composition or dosage form is administered or is for administration once-a-week. In an aspect, the composition or dosage form is administered or is for administration for a period of at least one week.

In an aspect, the above-mentioned treatment comprises the use/administration of the composition or dosage form described herein, in combination with one or more additional drugs. The combination of prophylactic/therapeutic compositions of the present disclosure may be administered or co-administered (*e.g.,* consecutively, simultaneously, at different times) in any conventional dosage form. Co-administration in the context of the present disclosure refers to the administration of more than one therapeutic in the course of a coordinated treatment to achieve an improved clinical outcome. Such co-administration may also be coextensive, that is, occurring during overlapping periods of time. For example, a first agent may be administered to a patient before, concomitantly, before and after, or after a second active agent is administered. The agents may in an aspect be combined/formulated in a single composition or dosage form and thus administered at the same time. In an aspect, the one or more active agent(s) is used/administered in combination with one or more agent(s) currently used to prevent or treat the disorder in question. For instance, the composition or dosage form described herein may be co-administered with at least one of agent used for the treatment of infectious diseases or related symptoms, *e.g.,* antibiotics, antivirals, fever medications (*e.g.,* acetaminophen, ibubrophen), etc.

### MODE(S) FOR CARRYING OUT THE INVENTION

The present disclosure is illustrated in further details by the following non-limiting examples.

### Example 1: Preparation of formulation 1 (IF5)

### Ingredients

| | **Amount/Unit** | **Ingredients** | **Amount/batch (200,000 capsules)** | **%** |
|---|---|---|---|---|
| | 250 mg | Propolis USP | 37.5 kg | 27.8% |
| | 150 mg | Tannic acid Sumac USP | 30 Kg | 16.7% |
| | 200 mg | Gum arabic USP | 40 Kg | 22.2% |
| | 150 mg | Cinnamon Powder USP | 30 kg | 16.7% |
| | 50 mg | Clove powder USP | 10 kg | 5.6% |
| | 6 mg | Magnesium stearate USP | 1.2 kg | 0.7% |
| | 94 mg | Microcrystalline cellulose M112 (Comprecel) USP | 18.8 kg | 10.4% |
| Total ingredients | 900 mg | | 167.5 kg | 100% |
| | 118 mg | #00 opaque Orange gelatin capsule | 22.4 kg | |
| Total weight | 1018 mg | | 189.9 Kg | |

### Procedure for preparation of capsules

All ingredients are passed through a 25-mesh screen, and mixed in a V blender according to the following sequence:
- Add Clove powder + Magnesium stearate + cinnamon powder
- Mix 5 minutes
- Add Propolis (Yellow wax) + gum arabic + Microcrystalline cellulose
- Mix 5 minutes
- Add Tannic Acid (Sumac powder)
- Mix 5 minutes

The mixture is then incorporated into opaque Orange gelatin capsules (900 mg/capsule) using an encapsulation apparatus equipped with a metal detector. Filled capsules are verified to ensure that they are properly closed and undamaged.

### Example 2: Preparation of formulation 2 (IF2)

### Ingredients

| | **Amount/Unit** | **Ingredients** | **Amount/batch (200,000 capsules)** | **%** |
|---|---|---|---|---|
| | 500 mg | Propolis USP | 100 kg | 62.5% |
| | 200 mg | Tannic acid Sumac USP | 40 Kg | 25% |
| | 6 mg | Magnesium stearate USP | 1.2 kg | 0.75% |
| | 94 mg | Microcrystalline cellulose M112 (Comprecel) USP | 18.8 kg | 11.75% |
| Total ingredients | 800 mg | | 160 kg | 100% |
| | 118 mg | #00 opaque Orange gelatin capsule | 22.4 kg | |
| Total weight capsule | 918 mg | | 182.4 Kg | |

### Procedure for preparation of capsules

The capsules are prepared according to the procedure described in Example 1, except that all ingredient (Propolis, Tannic Acid (Sumac), magnesium stearate and microcrystalline cellulose are mixed for 10 minutes, and 800 mg of the mixture is encapsulated.

### Example 3: Antiviral activity of the formulations

### Materials and Methods

*Formulations.* Formulation 1 (IF5) and formulation 2 (IF2) were dissolved in boiled distilled deionized water at 200 mg/ml. The water extracts were filtered using a Whatman No. 4 filter (cat. 1444 110) as a stock solution and diluted with culture medium to make various final concentrations for *in vitro* assays.

*Cell culture.* Hep-2 (human laryngeal carcinoma) and A549 (human type II alveolar pneumocyte cell) cell lines were obtained from American Type Culture Collection (ATCC^{®}, Rockville, MD). All cells were cultured in Eagle's modified essential medium (EMEM, Life Technologies, CA) supplemented with 10% fetal calf serum, 2 mM L-glutamine and penicillin/streptomycin at 37°C in 5% CO₂.

*Virus preparation and titration.* The A2 strain of RSV was purchased from the American Type Culture Collection (ATCC^{®}, VR-1401). The recombinant strain of RSV expressing green fluorescent protein (rgRSV; herein RSV-GFP) was a gift from Dr. M.E. Peeples (Children's Research Institute, Columbus, OH, USA). Hep-2 cells were used to amplify the viruses, as described previously (Das et al., 2020). For RSV infection, cells with 80% confluency in 12-well plates, were inoculated with the RSV virus and the plates were shaken several times gently during the adsorption period. After adsorption for 2 h at 37°C, the cells were washed with phosphate buffered saline (PBS), and 10% EMEM was added. The infection was allowed to proceed for 3 to 5 days until the entire monolayer shows cytopathic effects. The contents were resuspended in 1 ml aliquots, snap-frozen and stored at -80°C. Virus titration was done by plaque assay using Hep-2 cells in 24-well tissue culture plates and titers were maintained in the range of 10⁶ to 10⁸ pfu/ml for more than 6 months at -70°C. Lentivirus pseudotyped with SARS-CoV-2 spike protein was supplied by Creative Diagnostics (catalog number COV-PS02).

*Cytotoxicity assay.* Cytotoxicity of the formulations was examined by the growth inhibition of A549 cells. Briefly, A549 cells were seeded at a concentration of 2.5×10⁴ cells/well in 24-well plates and grown with 5% FCS-EMEM at 37°C for 2 days. The culture medium was replaced with fresh medium containing the formulations at various concentrations and the cells were grown for a further 3 days. The cells were treated with trypsin and the number of viable cells was determined by the trypan blue exclusion test. The 50% cytotoxic concentrations of the formulations reducing cell viability (CC₅₀) were determined from a curve relating the percentage of viable cells to the concentrations of the formulations. The antiviral capacity of the formulations was evaluated *in vitro* and compared to that exhibited by each of the formulations, as well as that of a mixture of them.

*Cytopathic effect-based antiviral assays.* Hep-2 were seeded in 24 tissue culture plates at 3x10⁵ /well. The formulations and Heparin were assayed for their ability to inhibit RSV infectivity. Heparin that is demonstrated to inhibit RSV infection was used in these experiments as previously described (Tripp *et al.,* 2001). Confluent cells at 70 to 80% were treated with the formulations at indicated concentrations at 37°C for 3 days. For RSV-GFP (green), A549 cells were infected at a multiplicity of infection (MOI) of 2, containing diluted compound. At 48 h pi, cells were washed, fixed with 4% paraformaldehyde (PAF) and examined by Zeiss microscopy.

For plaque reduction neutralization assays, cells were preincubated for 24 h at 37°C with the formulations (various doses up to 10 µg/ml), Heparin (5 µg/ml) or PBS followed by infection with RSV at a m.o.i. of 2 in the presence of diluted formulations. After 2 hours of incubation, cells were washed with PBS and overlaid with RPMI containing the same reagents (the formulations or Heparin), 10% FCS and 0.75% methylcellulose. Five days post-infection, the cells were fixed with 4% PAF and stained with Crystal violet. Plaques were counted under a dissecting microscope, and percent inhibition of virus infectivity of treated cells was determined versus untreated control wells (Feldman et al., 1999). Viral titers of Hep-2 cell cultures were measured also by the plaque method and expressed as plaque-forming units per well (PFU/well).

*Pseudovirus SARS-Cov-2 infection.* A549 cells (10⁴ cells) were seeded in 96 culture plate. At 24 h after cell seeding, cells were incubated in culture medium containing diluted formulations. Then cells were inoculated with concentrated SARS-CoV-2 pseudoviruses for 12 h in the presence of diluted formulations. After this incubation, pseudovirus containing media was replaced with regular EMEM media and incubated further for 48 h. Cells were fixed with 4% PAF for 10 min and washed with PBS. Images were taken by Confocal Zeiss LSM710 microscope (Oberkochen, Germany). All images were analyzed using ImageJ (Version 1.53a, National Institutes of Health, USA). SARS-CoV-2-GFP (green fluorescence), was measured and represented as relative fluorescence units (RFU). The total number of GFP expressing cells in each condition was normalized to the total number of cells in the respective wells.

### Results

The results depicted in **FIGs. 1A-D** show that formulation 1 and formulation 2, used separately or in combination, reduces infection of Hep-2 cells by RSV in a dose-dependent manner.

The results depicted in **FIGs. 2A-B** show that a mixture of formulation 1 and formulation 2 reduces infection of A549 cells by SARS-CoV-2 pseudoviruses in a dose-dependent manner.

Results from cytotoxicity assays show that the EC₅₀ values (concentration that reduced cell infection by 50%) were significantly lower than the CC₅₀ values (concentration that reduced cell viability by 50%) for formulation 1 (IF5), formulation 2 (IF2) and their combination (IF2/5) (P<0.05 by Student's t-test). For example, the EC₅₀ of IF2/5 is 7 ± 1.2, relative to a CC₅₀ of 68 ± 2.7.

### REFERENCES

Kujumgiev et al., J Ethnopharmacol 1999 Mar;64(3):235-40;
Sforcin et al., J. Ethnopharmacol. 2000;73:243-249;
Orsi et al., Journal of Venoms and Animals Toxins including Tropical Diseases, v. 6, n. 2., p. 205-219, 2000;
Orsi et al., Brazilian Journal of Microbiology, v. 37, n. 2, p. 108-112, 2000;
Scazzocchio et al., Microbiol Res. 2006;161(4):327-33);
Das et al., iScience. 2020 Jul 24; 23(7): 101256;
Feldman, S. A., Hendry, R. M. & Beeler, J. A. J. Virol. 73, 6610-6617 (1999).
Tripp et al., Nature Immunology volume 2, pages 732-738 (2001)

## Claims

1. A composition comprising from 20% to 75% (w/w) of propolis and from 10% to 30% (w/w) of tannic acid.

2. The composition of claim 1, wherein the composition comprises from 25% to 70% (w/w) of propolis.

3. The composition of claim 1 or 2, wherein the composition comprises from 10% to 25% (w/w) of tannic acid.

4. The composition of any one of claims 1 to 3, wherein the composition further comprises gum arabic, cinnamon and/or clove.

5. The composition of claim 4, wherein the composition comprises 10% to 30% (w/w) of gum arabic.

6. The composition of claim 4 or 5, wherein the composition comprises 10% to 30% (w/w) of cinnamon.

7. The composition of any one of claims 4 to 6, wherein the composition comprises 1% to 10% (w/w) of clove.

8. The composition of any one of claims 1 to 7, wherein the composition further comprises at least one carrier or excipient.

9. The composition of claim 8, wherein the at least one carrier or excipient comprises a binder and/or a lubricant.

10. The composition of claim 9, wherein the binder comprises a cellulose-based binder and/or the lubricant comprises magnesium stearate.

11. The composition of any one of claims 1 to 10, wherein the composition comprises:
| **Ingredients** | **% (w/w)** |
|---|---|
| Propolis | 50-70% |
| Tannic acid | 20-30% |
| Magnesium stearate | 0.5-2% |
| Microcrystalline cellulose | 5-15% |

12. The composition of any one of claims 1 to 10, wherein the composition comprises:
| **Ingredients** | **% (w/w)** |
|---|---|
| Propolis | 20-35% |
| Tannic acid | 14-20% |
| Gum arabic | 15-27% |
| Cinnamon powder | 10-20% |
| Clove powder | 2-8% |
| Magnesium stearate | 0.5-2% |
| Microcrystalline cellulose | 5-15% |

13. An oral dosage form comprising the composition of any one of claims 1 to 12.

14. The composition of any one of claims 1 to 12 or the dosage form of claim 13, for use in treating an infection in a subject.

15. The composition for use or dosage form for use according to claim 14, wherein the infection is an influenza infection, an RSV infection or a SARS-CoV-2 infection.

## Patentansprüche

1. Zusammensetzung, die 20 bis 75 Gew.-% Propolis und 10 bis 30 Gew.-% Tanninsäure enthält.

2. Die Zusammensetzung nach Anspruch 1, wobei die Zusammensetzung 25 bis 70 Gew.-% Propolis enthält.

3. Die Zusammensetzung nach Anspruch 1 oder 2, wobei die Zusammensetzung 10 bis 25 Gew.-% Tanninsäure enthält.

4. Die Zusammensetzung nach einem der Ansprüche 1 bis 3, wobei die Zusammensetzung zusätzlich Gummi arabicum, Zimt und/oder Nelken enthält.

5. Die Zusammensetzung nach Anspruch 4, wobei die Zusammensetzung 10 bis 30 Gew.-% Gummi arabicum enthält.

6. Die Zusammensetzung nach Anspruch 4 oder 5, wobei die Zusammensetzung 10 bis 30 Gew.-% Zimt enthält.

7. Die Zusammensetzung nach einem der Ansprüche 4 bis 6, wobei die Zusammensetzung 1 bis 10 Gew.-% Nelken enthält.

8. Die Zusammensetzung nach einem der Ansprüche 1 bis 7, wobei die Zusammensetzung ferner mindestens einen Träger oder Hilfsstoff enthält.

9. Die Zusammensetzung nach Anspruch 8, wobei der mindestens eine Träger oder Hilfsstoff ein Bindemittel und/oder ein Gleitmittel enthält.

10. Die Zusammensetzung nach Anspruch 9, wobei das Bindemittel ein Bindemittel auf Cellulosebasis und/oder das Gleitmittel Magnesiumstearat enthält.

11. Die Zusammensetzung nach einem der Ansprüche 1 bis 10, wobei die Zusammensetzung Folgendes umfasst:
| **Inhaltsstoffe** | **Gew.-%** |
|---|---|
| Propolis | 50 - 70% |
| Tanninsäure | 20 - 30% |
| Magnesiumstearat | 0,5 - 2% |
| mikrokristalline Cellulose | 5 - 15% |

12. Die Zusammensetzung nach einem der Ansprüche 1 bis 10, wobei die Zusammensetzung Folgendes umfasst:
| **Inhaltsstoffe** | **Gew.-%** |
|---|---|
| Propolis | 20 - 35% |
| Tanninsäure | 14 - 20% |
| Gummi arabicum | 15 - 27% |
| Zimtpulver | 10 - 20% |
| Nelkenpulver | 2-8% |
| Magnesiumstearat | 0,5 - 2% |
| mikrokristalline Cellulose | 5 - 15% |

13. Orale Darreichungsform, umfassend die Zusammensetzung nach einem der Ansprüche 1 bis 12.

14. Die Zusammensetzung nach einem der Ansprüche 1 bis 12 oder die Darreichungsform nach Anspruch 13 zur Verwendung bei der Behandlung einer Infektion bei einem Patienten.

15. Die Zusammensetzung zur Verwendung oder die Darreichungsform zur Verwendung nach Anspruch 14, wobei es sich bei der Infektion um eine Influenza-Infektion, eine RSV- Infektion oder eine SARS-CoV-2-Infektion handelt.

## Revendications

1. Composition comprenant de 20 % à 75 % (en poids/poids) de propolis et de 10 % à 30 % (en poids/poids) d'acide tannique.

2. Composition selon la revendication 1, laquelle composition comprend de 25 % à 70 % (en poids/poids) de propolis.

3. Composition selon la revendication 1 ou 2, laquelle composition comprend de 10 % à 25 % (en poids/poids) d'acide tannique.

4. Composition selon l'une quelconque des revendications 1 à 3, laquelle composition comprend en outre de la gomme arabique, de la cannelle et/ou du clou de girofle.

5. Composition selon la revendication 4, laquelle composition comprend 10 % à 30 % (en poids/poids) de gomme arabique.

6. Composition selon la revendication 4 ou 5, laquelle composition comprend 10 % à 30 % (en poids/poids) de cannelle.

7. Composition selon l'une quelconque des revendications 4 à 6, laquelle composition comprend 1 % à 10 % (en poids/poids) de clou de girofle.

8. Composition selon l'une quelconque des revendications 1 à 7, laquelle composition comprend en outre au moins un véhicule ou excipient.

9. Composition selon la revendication 8, dans laquelle l'au moins un véhicule ou excipient comprend un liant et/ou un lubrifiant.

10. Composition selon la revendication 9, dans laquelle le liant comprend un liant à base de cellulose et/ou le lubrifiant comprend du stéarate de magnésium.

11. Composition selon l'une quelconque des revendications 1 à 10, laquelle composition comprend :
| Ingrédients | % (en poids/poids) |
|---|---|
| Propolis | 50 à 70 % |
| Acide tannique | 20 à 30 % |
| Stéarate de magnésium | 0,5 à 2 % |
| Cellulose microcristalline | 5 à 15 % |

12. Composition selon l'une quelconque des revendications 1 à 10, laquelle composition comprend :
| Ingrédients | % (en poids/poids) |
|---|---|
| Propolis | 20 à 35 % |
| Acide tannique | 14 à 20 % |
| Gomme arabique | 15 à 27 % |
| Poudre de cannelle | 10 à 20 % |
| Poudre de clou de girofle | 2 à 8 % |
| Stéarate de magnésium | 0,5 à 2 % |
| Cellulose microcristalline | 5 à 15 %. |

13. Forme posologique à usage oral comprenant la composition de l'une quelconque des revendications 1 à 12.

14. Composition selon l'une quelconque des revendications 1 à 12 ou forme posologique selon la revendication 13, pour une utilisation dans le traitement d'une infection chez un sujet.

15. Composition pour une utilisation ou forme posologique pour une utilisation selon la revendication 14, dans laquelle l'infection est une infection grippale, une infection par RSV ou une infection par SARS-CoV-2.
